# EUROPEAN PATENT APPLICATION

(11) **EP 4 553 352 A1**
(43) Date of publication of application: **14.05.2025**
(21) Application number: 24210776.1
(22) Date of filing: 05.11.2024
(51) Int. Cl.: F16K 15/04, F16K 27/02, A01G 31/02, G01N 27/38, G01N 33/18, F16K 11/10

(54) **MEASUREMENT APPARATUS**

(30) Priority: 09.11.2023 JP 2023191412
(71) Applicant: SYNCA GROUP, LTD, 192-0045 Tokyo (JP)
(72) Inventor: Nakazawa, Hideta, Hachioji, Tokyo,, 192-0045 (JP)
(74) Representative: Bandpay & Greuter

(57) **Abstract**

A measurement apparatus includes a supply pipe that is supported by a top portion forming a fulcrum and is configured to be tilted to one side or the other side thereof, a cleaning liquid supply pipe that supplies distilled water to the supply pipe, a first valve that stops the flow of the distilled water from the supply pipe while the supply pipe is tilted to the one side, and a second valve disposed on the opposite side of the first valve from a supply port. While the supply pipe is not tilted, the first valve and second valve do not stop the flow of the distilled water from the supply pipe. While the supply pipe is tilted to the other side, the second valve prevents a liquid from flowing from the outside of the supply pipe toward the first valve.

## Description

### FIELD

The embodiments discussed herein relate to a measurement apparatus.

### BACKGROUND

Hydroponics have become increasingly popular. pH sensors for measuring the pH of an aqueous solution used in the hydroponics are known. For example, if a pH sensor whose surface is covered with a glass film is kept immersed in an aqueous solution, dust sticks to the glass film. To prevent this, it is preferable to immerse the pH sensor in the aqueous solution during measurement periods and not to immerse the pH sensor in the aqueous solution during non-measurement periods. In terms of this point, there is known a measurement apparatus that includes a base having a supporting portion forming a fulcrum, and a rod-shaped body having a supported portion supported by the supporting portion, the rod-shaped body including a measurement unit at one side thereof and a reservoir for storing a fluid at the other side thereof opposite to the one side with the supported portion therebetween. The rod-shaped body is balanced horizontally while the reservoir is filled with the fluid. While the first reservoir is not filled with the fluid, the rod-shaped body is tilted to the one side where the measurement unit is provided, so that the measurement unit is immersed in a solution. See, for example, Japanese Patent No. 7241444.

Japanese Patent No. 7241444 teaches that a pipe through which a cleaning liquid passes is arranged inside the rod-shaped body and that the cleaning liquid is supplied to the measurement unit through the pipe while the rod-shaped body is balanced horizontally. However, Japanese Patent No. 7241444 does not teach any specific method to achieve this feature.

### SUMMARY

According to one aspect, there is provided a liquid shut-off valve device including: a pipe that is supported by a fulcrum and is configured to be tilted to one side thereof or to another side thereof opposite to the one side; a supply port configured to supply a liquid to the pipe; a first valve configured to stop a flow of the liquid from the pipe in response to the pipe being tilted to the one side; and a second valve disposed on an opposite side of the first valve from the supply port, wherein each of the first valve and the second valve includes a spherical-shaped object and a cylinder having openings at both ends thereof, the openings having diameters smaller than a diameter of the spherical-shaped object, wherein the first valve and the second valve do not stop the flow of the liquid from the pipe while the pipe is not tilted, wherein the spherical-shaped object of the first valve engages with the cylinder of the first valve to stop the flow of the liquid from the pipe in response to the pipe being tilted to the one side, and wherein the spherical-shaped object of the second valve engages with the cylinder of the second valve to prevent a liquid from flowing from an outside of the pipe toward the first valve in response to the pipe being tilted to the other side.

### BRIEF DESCRIPTION OF DRAWINGS

FIG. 1 illustrates a measurement apparatus according to a first embodiment.
FIG. 2 is a view for describing the operation of a first mechanical part according to the embodiment.
FIGS. 3 to 5 are views for describing the operation of the measurement apparatus according to the embodiment.
FIG. 6 illustrates a measurement apparatus according to a second embodiment.
FIG. 7 is a view for describing the configuration of a liquid shut-off valve device according to the embodiment.
FIG. 8 is a view for describing how the liquid shut-off valve device operates when the left side of a rod-shaped body rises and the right side thereof goes down.
FIG. 9 is a view for describing how the liquid
shut-off valve device operates when the right side of the rod-shaped body rises and the left side thereof goes down.

### DESCRIPTION OF EMBODIMENTS

Hereinafter, a measurement apparatus according to embodiments will be described with reference to the accompanying drawings.

For easy understanding of the embodiments, the positions, sizes, shapes, ranges, and others of the individual components illustrated in the drawings and the like may not represent the actual positions, sizes, shapes, ranges, and others. Therefore, the embodiments are not limited to the positions, sizes, shapes, ranges, and others disclosed in the drawings and the like.

Elements that are each expressed in a singular form in the embodiments may be plural in use, expect otherwise particularly specified in writing.

### (First Embodiment)

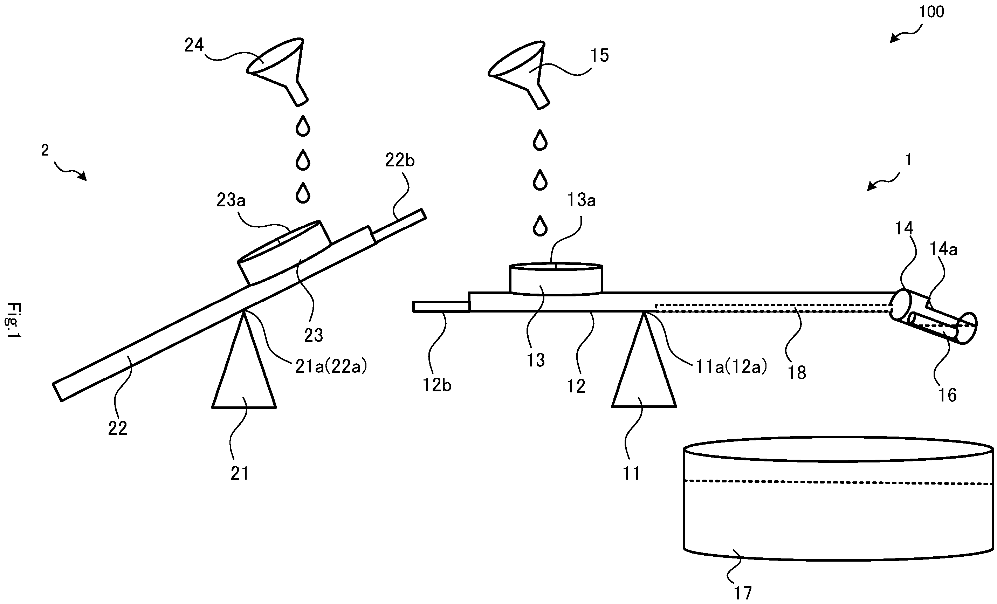

FIG. 1 illustrates a measurement apparatus according to a first embodiment.

The measurement apparatus 100 of the first embodiment includes a first mechanical part 1 and a second mechanical part 2.

### (First mechanical part)

The first mechanical part 1 includes a base 11 and a rod-shaped body 12.

The base 11 has a top portion 11a that forms a supporting portion (fulcrum), and a supported portion 12a of the rod-shaped body 12 is placed on the supporting portion. In the present embodiment, the base 11 and rod-shaped body 12 are illustrated schematically. How to engage the base 11 and the rod-shaped body 12 with each other is not limited to a particular engagement method, but for example, an engagement method based on a bamboo rocking water fountain mechanism called Shishi-odoshi or a seesaw mechanism may be employed.

A reservoir 13 is provided at one side of the rod-shaped body 12, and an engaged portion 12b is provided at the one-side end of the rod-shaped body 12. In addition, a sensor mounting portion 14 is provided at the other-side end of the rod-shaped body 12 opposite to the one side with the supported portion 12a therebetween.

The reservoir 13 has an opening 13a. A liquid (for example, water) discharged from a liquid discharge part 15 flows into the reservoir 13 through the opening 13a. The reservoir 13 stores the liquid flowing therein.

A pH sensor (measurement unit) 16 is mounted in the sensor mounting portion 14. The pH sensor 16 detects the pH of a liquid stored in a water tank 17, for example. The surface of the pH sensor 16 of the embodiment is covered with a glass film. The pH sensor 16 has a short range communication means so as to send information on the detected pH to a computer, not illustrated. Examples of short range communication include Wi-Fi and Bluetooth (registered trademark).

In addition, an opening (cutout) 14a is formed in the sensor mounting portion 14.

A supply pipe 18 is arranged inside the rod-shaped body 12. A cleaning liquid (for example, distilled water) is supplied from a supply means, not illustrated, to the supply pipe 18. The cleaning liquid supplied to the supply pipe 18 is then supplied to the sensor mounting portion 14. The dotted line illustrated in the sensor mounting portion 14 indicates the water level of the cleaning liquid supplied to the sensor mounting portion 14. The cleaning liquid is supplied to the detection part of the pH sensor 16 to thereby clean the detection part of the pH sensor 16.

The water tank 17 is filled with a solution (nutrient solution). For example, crops to be grown in hydroponics are placed in this water tank 17.

The operation of this first mechanical part 1 will be described simply.

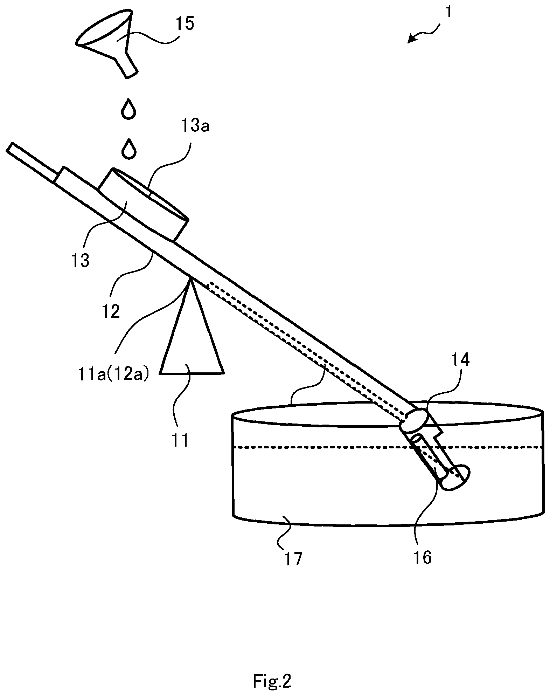

FIG. 2 is a view for describing the operation of the first mechanical part according to the embodiment.

In the following description, the left part of the rod-shaped body 12 from the top portion 11a is referred to as the "left side of the rod-shaped body 12," and the right part of the rod-shaped body 12 is referred to as the "right side of the rod-shaped body 12."

When the reservoir 13 is not filled with the liquid, the right side of the rod-shaped body 12 goes down, so that part or all of the sensor mounting portion 14 is positioned in the water tank 17, as illustrated in FIG. 2. In this state, the solution stored in the water tank 17 flows from the opening 14a into the sensor mounting portion 14, so that the pH sensor 16 is immersed in the solution and is thus able to measure the pH of the solution in the water tank 17. It may be so designed as not to cause the sensor mounting portion 14 to come into contact with the bottom surface of the water tank 17 using the buoyancy of the rod-shaped body 12 and the sensor mounting portion 14. Alternatively, it may be so designed as to cause the sensor mounting portion 14 to come into contact with the bottom surface of the water tank 17 gently using the resistance of the solution in the water tank 17.

As the reservoir 13 becomes filled with the liquid discharged from the liquid discharge part 15, the left side of the rod-shaped body 12 gradually goes down, and accordingly the right side thereof rises. In this connection, as the left side of the rod-shaped body 12 goes down, the position of the opening 13a gradually moves to the left in FIG. 2. Note that the opening 13a is formed large enough to receive the liquid discharged from the liquid discharge part 15. Then, when the reservoir 13 is filled with the liquid, the rod-shaped body 12 is balanced horizontally and remains in equilibrium, as illustrated in FIG. 1. While the liquid continues to be discharged from the liquid discharge part 15, any excess liquid that the reservoir 13 is not able to store spills from the reservoir 13, thereby keeping the rod-shaped body 12 in equilibrium.

It is possible to adjust the period of time during which the pH sensor 16 is placed in the water tank 17, by controlling the amount of the liquid discharged from the liquid discharge part 15 or by changing the capacity of the reservoir 13. In the case where the measurement time of the pH sensor 16 is 5 minutes, for example, the amount of the liquid discharged from the liquid discharge part 15 may be adjusted so that the reservoir 13 is filled with the liquid in 5 minutes.

Refer now back to FIG. 1.

### (Second Mechanical Part)

The second mechanical part 2 is configured using the principal of the bamboo rocking water fountain called "Shishi-odoshi."

The second mechanical part 2 includes a base 21 and a rod-shaped body 22.

The base 21 has a top portion 21a that forms a supporting portion (fulcrum), and a supported portion 22a of the rod-shaped body 22 is placed on the supporting portion. In the present embodiment, the base 21 and the rod-shaped body 22 are illustrated schematically. How to engage the base 21 and the rod-shaped body 22 with each other is not limited to a particular engagement method, but for example, an engagement method based on a bamboo rocking water fountain mechanism called Shishi-odoshi or a seesaw mechanism may be employed.

A reservoir 23 is provided at one side of the rod-shaped body 22 away from the top portion 21a, and an engaging portion 22b is provided at the one-side end of the rod-shaped body 22.

The reservoir 23 has an opening 23a. A liquid (for example, water) discharged from a liquid discharge part 24 flows into the reservoir 23 through the opening 23a. The reservoir 23 stores the liquid flowing therein.

In the following description, the left part of the rod-shaped body 22 from the top portion 21a is referred to as the "left side of the rod-shaped body 22," and the right part of the rod-shaped body 22 is referred to as the "right side of the rod-shaped body 22."

In the second mechanical part 2, when the amount of the liquid stored in the reservoir 23 is less than or equal to a predetermined amount, the left side of the rod-shaped body 22 goes down, and accordingly the right side of the rod-shaped body 22 rises, as illustrated in FIG. 1.

As the reservoir 23 becomes filled with the liquid discharged from the liquid discharge part 24, the right side of the rod-shaped body 22 gradually goes down, and accordingly the left side thereof rises. In this connection, as the right side of the rod-shaped body 22 goes down, the position of the opening 23a gradually moves to the right in FIG. 1. Note that the opening 23a is formed large enough to receive the liquid discharged from the liquid discharge part 24.

The following describes the operation of the measurement apparatus 100.

As described earlier, when the reservoir 13 is filled with the liquid, the rod-shaped body 12 is balanced horizontally and remains in equilibrium, as illustrated in FIG. 1. While the liquid continues to be discharged from the liquid discharge part 15, any excess liquid that the reservoir 13 is not able to store spills from the reservoir 13, thereby keeping the rod-shaped body 12 in equilibrium. At this time, the cleaning liquid is supplied from a supply means, not illustrated, to the supply pipe 18. The cleaning liquid supplied to the supply pipe 18 is then supplied to the sensor mounting portion 14.

As the reservoir 23 becomes filled with the liquid discharged from the liquid discharge section 24, the right side of the rod-shaped body 22 goes down with the momentum gradually increasing, and accordingly the left side of the rod-shaped body 22 rises.

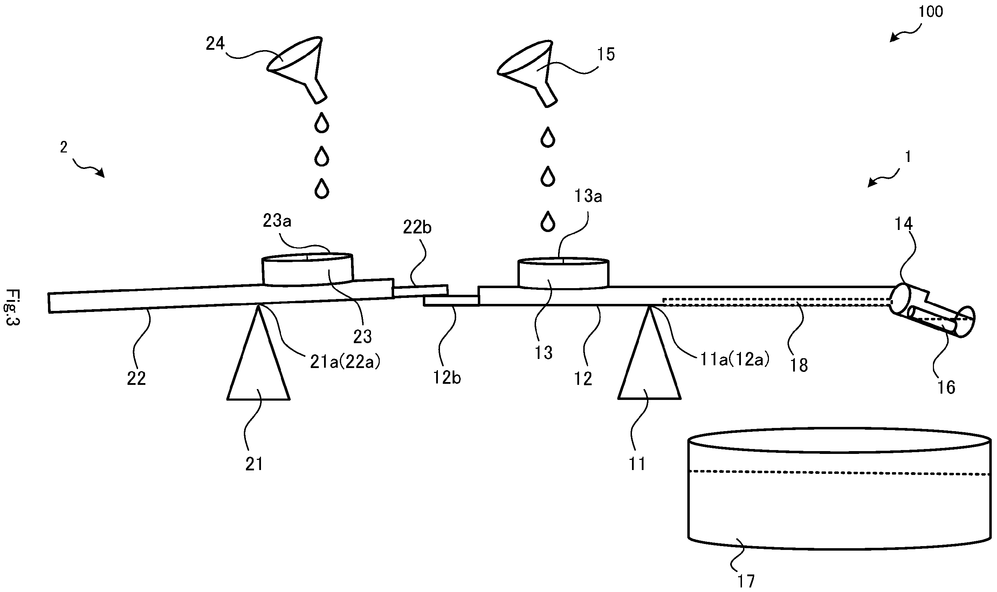

FIGS. 3 to 5 are views for describing the operation of the measurement apparatus according to the embodiment.

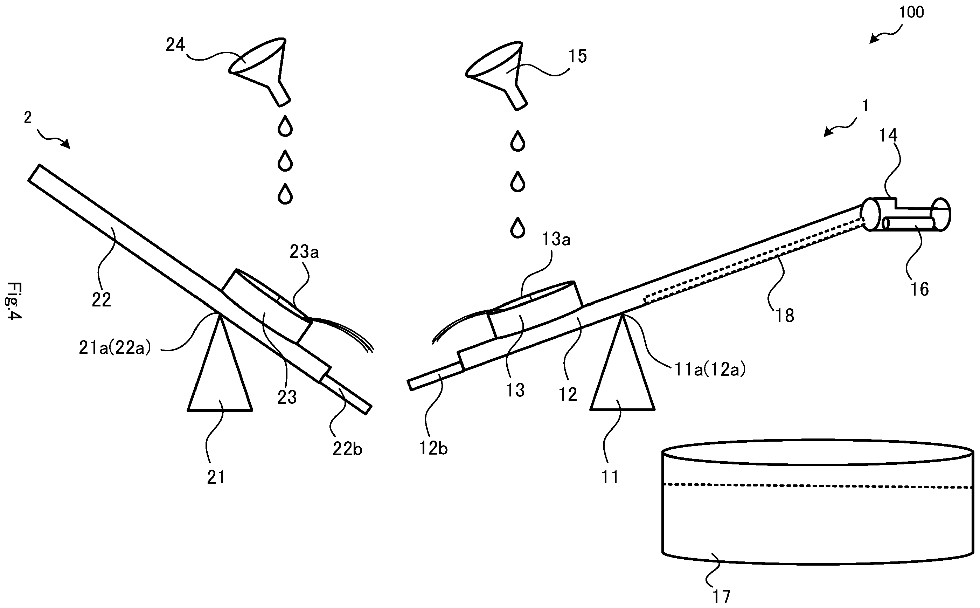

As illustrated in FIG. 3, when the right side of the rod-shaped body 22 goes down, the engaging portion 22b bumps into the engaged portion 12b and presses the engaged portion 12b downward in FIG. 3. Then, the left side of the rod-shaped body 12 goes down with great momentum, so that the liquid stored in the reservoir 13 spills from the opening 13a, as illustrated in FIG. 4. As a result, the left side of the rod-shaped body 12 becomes lighter than the right side of the rod-shaped body 12. In addition, after pressing the engaged portion 12b, the right side of the rod-shaped body 22 goes down as well, so that the liquid stored in the reservoir 23 spills from the opening 23a. As a result, the right side of the rod-shaped body 22 becomes lighter than the left side of the rod-shaped body 22. In this connection, it may be so designed that the liquid stored in the sensor mounting portion 14 is discharged to the outside through the supply pipe 18 at this time.

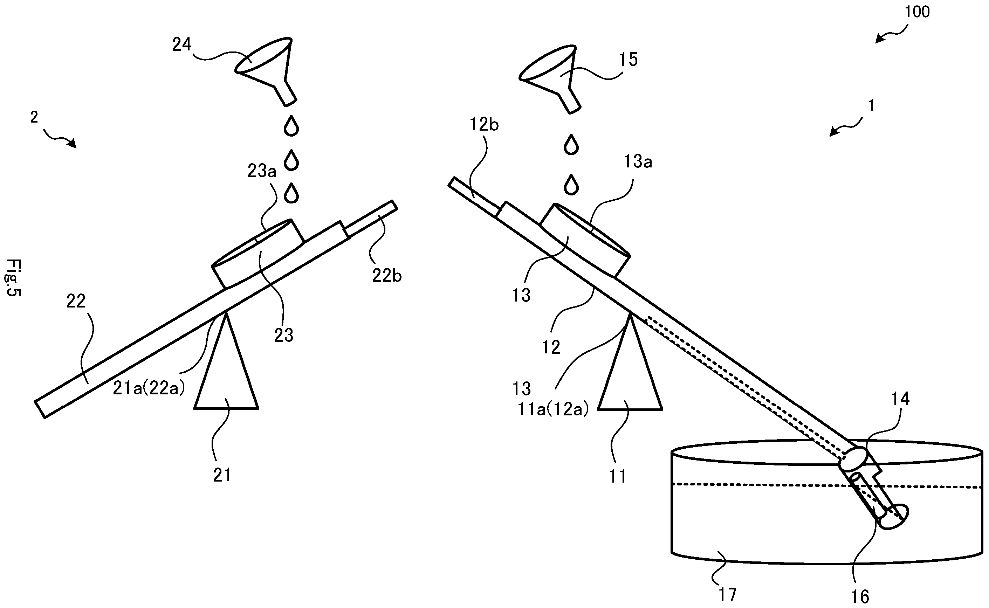

After that, the left side of the rod-shaped body 12 rises, and accordingly the right side of the rod-shaped body 12 goes down, as illustrated in FIG. 5. Therefore, the pH sensor 16 is placed in the water tank 17, so that the pH sensor 16 is able to measure the pH of the liquid in the water tank 17. In addition, the right side of the rod-shaped body 22 rises, and accordingly the left side of the rod-shaped body 22 goes down.

After that, the liquid starts to be stored in both the reservoir 13 and the reservoir 23. The flow rates of the liquid discharge parts 15 and 24, the positions of the reservoirs 13 and 23, and others are adjusted so that the reservoir 13 starts to go down before the reservoir 23 starts to go down. With such adjustments, the state is returned back to that illustrated FIG. 1 again.

As described above, the measurement apparatus 100 of the embodiment includes the base 11 with the top portion 11a forming a fulcrum, and the rod-shaped body 12 having the supported portion 12a supported by the top portion 11a and including the pH sensor 16 provided at one side thereof and the reservoir 13 provided at the other side opposite to the one side with the supported portion 12a therebetween. The reservoir 13 is configured to store a liquid. When the reservoir 13 is filled with the liquid, the rod-shaped body 12 is balanced horizontally. When the reservoir 13 is not filled with the liquid, the rod-shaped body 12 is tilted to the side where the pH sensor 16 is provided, so that the pH sensor 16 is immersed in the solution of the water tank 17. With the above configuration, when the measurement by the pH sensor 16 is not performed, the reservoir 13 is filled with the liquid so as to keep the rod-shaped body 12 balanced horizontally. That is, it is possible to prevent dust from sticking to the glass film of the pH sensor 16 due to the pH sensor 16 being left immersed in the water tank 17.

In addition, when the rod-shaped body 12 is tilted to the side where the reservoir 13 is provided, the liquid stored in the reservoir 13 flows out of the reservoir 13, and thus the rod-shaped body 12 is tilted to the side where the pH sensor 16 is provided. This configuration makes it easy to manage the position of the pH sensor 16.

In addition, a power unit (second mechanical part 2 in the embodiment) is provided, which tilts the rod-shaped body 12 to the side where the reservoir 13 is provided. By operating the second mechanical part 2 at desired timing, it becomes possible to manage the timing of the measurement using the pH sensor 16 easily.

In addition, the supply pipe 18 for allowing the cleaning liquid to pass through is provided inside the rod-shaped body 12 so that the cleaning liquid is supplied to the pH sensor 16 through the supply pipe 18 while the rod-shaped body 12 is balanced horizontally. By doing so, the pH sensor 16 is cleaned.

In addition, the sensor mounting portion 14 is provided, which has the opening 14a, allows the pH sensor 16 to be mounted therein, and stores the cleaning liquid to be supplied to the pH sensor 16. Therefore, the sensor mounting portion 14 is able to store the cleaning liquid supplied from the supply pipe 18, which makes it possible to clean the glass film of the pH sensor 16 more reliably. In addition, the cleaning liquid is discharged to the water tank 17 when the measurement using the pH sensor 16 is performed. Therefore, it is easy to replace the cleaning liquid.

In the embodiment, the liquid is stored in the reservoir 13. Alternatively, gas (fluid) that is heavier than air may be stored in the reservoir 13.

Further, in the embodiment, as the power unit that tilts the rod-shaped body 12 to the side where the reservoir 13 is provided, the second mechanical part 2 is used. The power unit is not limited thereto, and for example, a motor is provided in the vicinity of the top portion 11a, and the rod-shaped body 12 may be tilted to the side where the reservoir 13 is provided by rotating the motor. Alternatively, the rod-shaped body 12 may be tilted to the side where the reservoir 13 is provided by using wind power.

Still further, in the embodiment, the pH of the solution (nutrient solution) in the water tank 17 in which crops for the hydroponics are placed is measured. The measurement target is not limited thereto and for example, water-soluble cutting fluids for cutting (for example, soluble type, emulsion type, chemical solution type, and others) may be used.

### (Second Embodiment)

A measurement apparatus according to a second embodiment will now be described.

In the following description, the measurement apparatus of the second embodiment will be described by focusing on different features from the measurement apparatus of the first embodiment described above, and the description on the same features will be omitted.

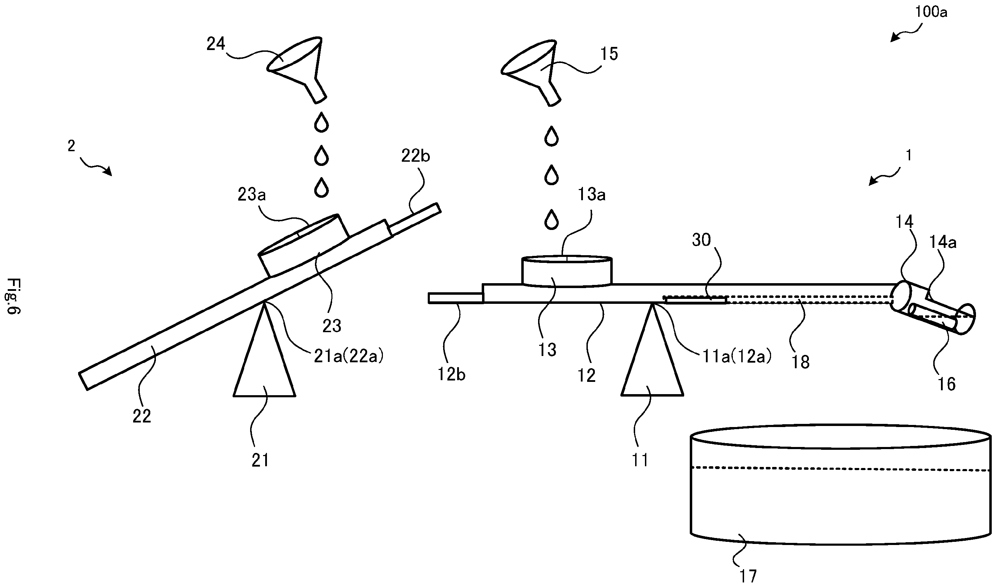

FIG. 6 illustrates a measurement apparatus according to the second embodiment.

The measurement apparatus 100a of the second embodiment illustrated in FIG. 6 has the same configuration as the measurement apparatus 100 of the first embodiment except that the measurement apparatus 100a includes a liquid shut-off valve device 30 that is a specific implementation of the configuration of the supply means of supplying a cleaning liquid to the supply pipe 18.

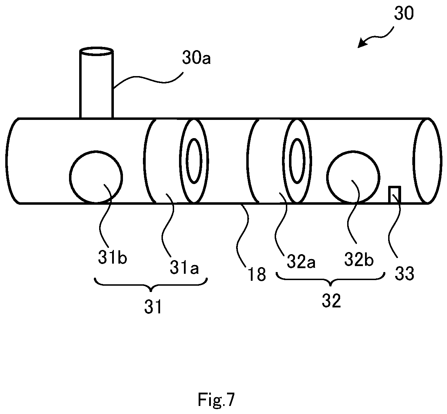

FIG. 7 is a view for describing the configuration of the liquid shut-off valve device according to the embodiment.

The liquid shut-off valve device 30 includes a cleaning liquid supply pipe 30a, a first valve 31, a second valve 32, and a stopper 33.

The cleaning liquid supply pipe 30a is configured to supply a cleaning liquid (for example, distilled water) to the supply pipe 18.

The first valve 31, second valve 32, and stopper 33 are arranged inside the supply pipe 18. The sensor mounting portion 14 and pH sensor 16, which are arranged on the right side of the stopper 33, are not illustrated in FIG. 7.

The first valve 31 includes a hole valve 31a and a ball 31b.

The hole valve 31a has a cylindrical shape with openings at both ends thereof. The ball 31b is a spherical-shaped object. The diameters of the openings of the hole valve 31a are smaller than the diameter of the ball 31b. The hole of the hole valve 31a between the openings is tapered such that the diameter of the hole decreases from the side (outer side) that the ball 31b contacts toward the inner side. When the ball 31b is pressed against the hole valve 31a, the ball 31b and hole valve 31a function as a liquid shut-off valve that does not allow the passage of a fluid.

The second valve 32 includes a hole valve 32a and a ball 32b. The second valve 32 has the same configuration as the first valve 31. More specifically, the hole valve 32a has a cylindrical shape with openings at both ends thereof. The ball 32b has a spherical-shaped object. The diameters of the openings of the hole 32a are smaller than the diameter of the ball 32b. The hole of the hole valve 32a between the openings is tapered such that the diameter of the hole decreases from the side (outer side) that the ball 32b contacts toward the inner side. When the ball 32b is pressed against the hole valve 32a, the ball 32b and hole valve 32a function as a liquid shut-off valve that does not allow the passage of a flid.

The stopper 33 prevents the ball 32b from being away a predetermined distance or longer from the hole valve 32a.

In this connection, the positions of the hole valve 31a and hole valve 31b inside the supply pipe 18, the distance between the hole valve 31a and the hole valve 31b, the distance between the cleaning liquid supply pipe 30a and the hole valve 31a, and the shape and structure of the stopper 33 are not limited to those illustrated.

The following describes the operation of the liquid shut-off valve device 30.

As described in the first embodiment, when the reservoir 13 is filled with a liquid, the rod-shaped body 12 is balanced horizontally and remains in equilibrium, as illustrated in FIG. 6. Although the liquid continues to be discharged from the liquid discharge part 15, any excess liquid that the reservoir 13 is not able to store spills from the reservoir 13, thereby keeping the rod-shaped body 12 in equilibrium. At this time, a cleaning liquid is supplied from the cleaning liquid supply pipe 30a to the supply pipe 18. The cleaning liquid supplied to the supply pipe 18 is then supplied to the sensor mounting portion 14 through the hole valves 31a and 32a. The distilled water fills up to the maximum capacity in the supply pipe 18 and sensor mounting portion 14. Note that it is not a problem for the distilled water to spill from the opening 14a.

The following describes how the liquid shut-off valve device 30 operates when the left side of the rod-shaped body 12 rises and the right side of the rod-shaped body 12 goes down as illustrated in FIG. 5.

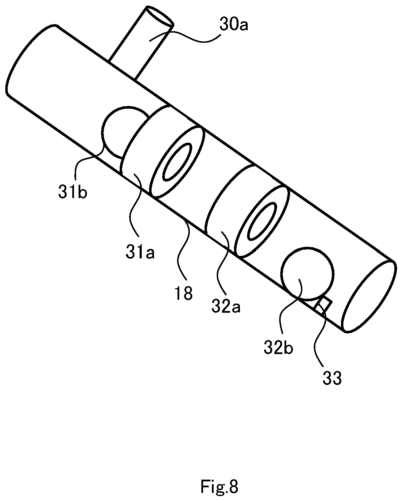

FIG. 8 is a view for describing how the liquid shut-off valve device operates when the left side of the rod-shaped body rises and the right side thereof goes down.

As described in the first embodiment, when the pH sensor 16 is placed in the water tank 17, the pH sensor 16 is able to measure the pH of the solution in the water tank 17. At this time, the distilled water that has filled the supply pipe 18 and sensor mounting portion 14 spills into the water tank 17. In addition, the ball 31b comes into contact with the opening of the hole valve 31a to block the opening of the hole valve 31a. This prevents the distilled water from flowing from the cleaning liquid supply pipe 30a toward the right side of the hole valve 31a. Since the hole valve 32a and the ball 32b do not contact each other, the supply pipe 18 on the right side of the hole valve 31a is empty. Therefore, the pH sensor 16 is able to measure the pH correctly with little influence from the distilled water. It is possible to adjust the measurement time of the pH sensor 16 on the basis of the accuracy of the pH sensor 16 by controlling the amount of the liquid discharged from the liquid discharge part 15 to the reservoir 13.

After that, the liquid starts to be stored in the reservoir 13. Then, when the reservoir 13 is filled with the liquid, the rod-shaped body 12 is balanced horizontally and remains in equilibrium, as illustrated in FIG. 6. Thereby, the distilled water fills up to the maximum capacity in the supply pipe 18 and sensor mounting portion 14. The pH sensor 16 is immersed into the distilled water, thereby preventing the pH sensor 16 from getting dry.

The following describes how the liquid shut-off valve device 30 operates when the right side of the rod-shaped body 12 rises and the left side of the rod-shaped body 12 goes down as illustrated in FIG. 4.

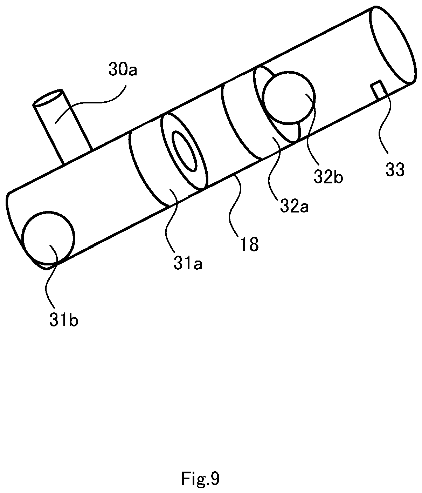

FIG. 9 is a view for describing how the liquid shut-off valve device operates when the right side of the rod-shaped body rises and the left side thereof goes down.

In this state, the ball 32b comes into contact with the opening of the hole valve 32a to block the opening of the hole valve 32a. This prevents the liquid remaining in the sensor mounting portion 14, which has flowed in from the water tank 17, from flowing to the left side of the hole valve 32a.

As described above, the measurement apparatus 100a of the second embodiment includes the supply pipe 18 that is supported by the top portion 11a serving as a fulcrum and is configured to be tilted to one side or the other side, the cleaning liquid supply pipe 30a configured to supply the distilled water to the supply pipe 18, the first valve 31 that is configured to stop the flow of the distilled water from the supply pipe 18 when the supply pipe 18 is tilted to the one side, and the second valve 32 disposed on the opposite side of the first valve 31 from a supply port when the supply pipe 18 is tilted to the one side. When the supply pipe 18 is not tilted, the first valve 31 and second valve 32 do not stop the flow of the distilled water from the supply pipe 18. When the supply pipe 18 is tilted to the other side, the second valve 32 prevents the liquid from flowing from the outside of the supply pile 18 toward the first valve 31.

The measurement apparatus 100a of the second embodiment provides the same effects as the measurement apparatus 100 of the first embodiment.

In addition, the measurement apparatus 100a of the second embodiment makes it possible to automate the supply of distilled water with a simple configuration.

Heretofore, the measurement apparatus according to the present disclosure has been described with respect to the embodiments illustrated. The configuration is not limited thereto, and the components of each unit may be replaced with other components having equivalent functions. In addition, other desired configurations and steps may be added to the embodiments.

In addition, desired two or more configurations (features) in the above-described embodiments may be combined.

According to one aspect, it is possible to automate the supply of a cleaning liquid with a simple configuration.

## Claims

1. A liquid shut-off valve device comprising:
a pipe that is supported by a fulcrum and is configured to be tilted to one side thereof or to an other side thereof opposite to the one side;
a supply port configured to supply a liquid to the pipe;
a first valve configured to stop a flow of the liquid from the pipe in response to the pipe being tilted to the one side; and
a second valve disposed on an opposite side of the first valve from the supply port,
wherein each of the first valve and the second valve includes a spherical-shaped object and a cylinder having openings at both ends thereof, the openings having diameters smaller than a diameter of the spherical-shaped object,
wherein the first valve and the second valve do not stop the flow of the liquid from the pipe while the pipe is not tilted,
wherein the spherical-shaped object of the first valve engages with the cylinder of the first valve to stop the flow of the liquid from the pipe in response to the pipe being tilted to the one side, and
wherein the spherical-shaped object of the second valve engages with the cylinder of the second valve to prevent a liquid from flowing from an outside of the pipe toward the first valve in response to the pipe being tilted to the other side.

2. A measurement apparatus comprising:
a base having a supporting portion forming a fulcrum; and
a rod-shaped body having a supported portion supported by the supporting portion, the rod-shaped body including a measurement unit at one side thereof and a reservoir for storing a fluid at an other side thereof opposite to the one side with the supported portion therebetween,
wherein the rod-shaped body is balanced horizontally while the reservoir is filled with the fluid,
wherein, while the reservoir is not filled with the fluid, the rod-shaped body is tilted to the one side where the measurement unit is provided, so that the measurement unit is immersed in a solution,
wherein a pipe through which a cleaning liquid passes is disposed inside the rod-shaped body,
wherein the measurement apparatus further includes a supply port configured to supply a liquid to the pipe, a first valve configured to stop a flow of the liquid from the pipe in response to the pipe being tilted to the one side where the measurement unit is provided, and a second valve disposed on an opposite side of the first valve from the supply port,
wherein each of the first valve and the second valve includes a spherical-shaped object and a cylinder having openings at both ends thereof, the openings having diameters smaller than a diameter of the spherical-shaped object,
wherein the first valve and the second valve do not stop the flow of the liquid from the pipe while the rod-shaped body is balanced horizontally, so that the cleaning liquid is supplied to the measurement unit through the pipe,
wherein the spherical-shaped object of the first valve engages with the cylinder of the first valve in response to the pipe being tilted to the one side where the measurement unit is provided, so as to stop the flow of the liquid from the pipe, and
wherein the spherical-shaped object of the second valve engages with the cylinder of the second valve in response to the pipe being tilted to the other side where the reservoir is provided, so as to prevent a liquid from flowing from an outside of the pipe toward the first valve.
